# EUROPÄISCHE PATENTSCHRIFT

(11) **EP 2 428 208 B1**
(45) Veröffentlichungstag und Bekanntmachung des Hinweises auf die Patenterteilung: **30.11.2016**
(21) Anmeldenummer: 11008821.8
(22) Anmeldetag: 31.10.2007
(51) Int. Cl.: G01N 33/50

(54) **Verfahren zur Bestimmung des Potentials von pharmazeutischen Substanzen, den zellulären Glutathion-Gehalt zu senken und den zellulären ROS-Gehalt zu steigern**
Method of determining the potency of pharmaceutical substances to reduce the cellular glutathion content and to increase the cellular ROS content
Procédé de détermination la puissance de substances pharmaceutiques réduisant la teneur cellulaire en glutathion et augmentant la teneur cellularire en ROS

(30) Priorität: 29.11.2006 DE 102006058183
(43) Veröffentlichungstag der Anmeldung: 14.03.2012
(62) Teilanmeldung aus: 07819467.7
(73) Patentinhaber: Eberhard-Karls-Universität Tübingen Universitätsklinikum, 72076 Tübingen (DE)
(72) Erfinder: Röcken, Martin, 72076 Tübingen (DE); Ghoreschi, Kamran, 26131 Oldenburg (DE); Weigert, Christina Mathilde, 72076 Tübingen (DE)
(74) Vertreter: Witte, Weller & Partner Patentanwälte mbB

(56) Entgegenhaltungen:
- EP-A1- 1 354 601
- WO-A1-00/48632
- WO-A2-01/68069
- WO-A2-02/100351
- WO-A2-2004/003565
- US-A- 5 994 402
- US-A1- 2005 079 502
- US-A1- 2005 101 646
- KARG E ET AL: "Modulation of Glutathione Level in Cultured Human Melanoma Cells", ACTA DERMATO-VENEREOLOGICA, Bd. 69, Nr. 2, 1989, Seiten 137-141, XP008153962,
- KARG E ET AL: "Alteration of Glutathione Level in Human Melanoma Cells: Effect of N-Acetyl-L-Cysteine and its Analogues", PIGMENT CELL RESEARCH, Bd. 3, 1990, Seiten 11-15, XP002680970,
- UTSUGI M ET AL: "c-Jun N-Terminal Kinase Negatively Regulates Lipopolysaccharide-Induced IL-12 Production in Human Macrophages", THE JOURNAL OF IMMUNOLOGY, Bd. 171, 2003, Seiten 628-635, XP002680971,
- BEKRIS L M ET AL: "Glutathione-S-transferase M1 and T1 polymorphisms and associations with type 1 diabetes age-at-onset", AUTOIMMUNITY, INFORMA HEALTHCARE, GB, Bd. 38, Nr. 8, 1. Dezember 2005 (2005-12-01), Seiten 567-575, XP008119128, ISSN: 0891-6934, DOI: 10.1080/08916930500407238
- SUTHANTHIRAN M ET AL: "GLUTATHIONE REGULATES ACTIVATION-DEPENDENT DNA SYNTHESIS IN HIGHLY PURIFIED NORMAL HUMAN T LYMPHOCYTES STIMULATED VIA THE CD2 AND CD3 ANTIGENS", PROCEEDINGS OF THE NATIONAL ACADEMY OF SCIENCES, NATIONAL ACADEMY OF SCIENCES, US, vol. 87, no. 9, 1 May 1990 (1990-05-01), pages 3343-3347, XP002283266, ISSN: 0027-8424, DOI: 10.1073/PNAS.87.9.3343
- S. SCHILLING ET AL: "Fumaric acid esters are effective in chronic experimental autoimmune encephalomyelitis and suppress macrophage infiltration.", CLINICAL AND EXPERIMENTAL IMMUNOLOGY, vol. 145, 2006, pages 101-107,

## Beschreibung

Die vorliegende Erfindung betrifft Verfahren zur Bestimmung des Potentials von pharmazeutischen Substanzen, den zellulären Glutathiongehalt zu senken und den zellulären ROS-Gehalt zu steigern, um damit das Potential der pharmazeutischen Substanz zu bestimmen, zur Therapie von T-Zell vermittelten Autoimmunkrankheiten eingesetzt zu werden.

Autoimmunität beruht auf einer spezifischen, adaptiven Immunantwort gegen körpereigene Antigene. Normalerweise lässt das Immunsystem körpereigene Substanzen unbehelligt und bekämpft nur Fremdkörper. Autoimmunität lässt sich als Ergebnis eines Zusammenbruchs der Toleranz gegenüber körpereigenen Stoffen und/oder eines defekten Kontroll- und Regulationsmechanismus des Immunsystems auffassen. Bisher sind die genauen Ursachen für die Entstehung von Autoimmunkrankheiten zwar unbekannt, es wird jedoch vermutet, dass neben umweltbedingten auch erbliche Faktoren eine Rolle spielen. T-Lymphozyten scheinen an der Auslösung der Krankheit wesentlich beteiligt zu sein, da sie sowohl als zytotoxische T-Lymphozyten als auch durch die Aktivierung von Makrophagen Gewebeschädigungen verursachen können.

Autoimmunkrankheiten lassen sich in gewebe- oder organspezifische sowie systemische, also nicht-organspezifisch Autoimmunkrankheiten einteilen. So ist beispielsweise die Erkrankung Multiple Sklerose ein Beispiel für eine organspezifische, T-Zell vermittelte Autoimmunerkrankung beim Menschen.

In der Praxis ist das therapeutische Vorgehen durch den chronischen, langsam fortschreitenden Charakter der meisten Autoimmunkrankheiten limitiert. Zu Teilerfolgen führte der Einsatz von Immunsuppressiva und Corticosteroiden.

An Hand des Cytokinprofils von CD4⁺ T-Helfer(T_{H})-Zellen lässt sich der Verlauf chronischer Autoimmunkrankheiten, Allergien oder Infektionen voraussagen. Die T_{H}-Lymphozyten werden u.a. auf der Basis der von ihnen produzierten Zytokine weiter unterteilt: Während T_{H}1 Zellen Interferon γ (IFN-γ) und Interleukin-2 (IL-2) synthetisieren, produzieren T_{H}2 Zellen stattdessen Interleukin-4 (IL-4), Interleukin-5 (IL-5), Interleukin-6 (IL-6), Interleukin-9 (IL-9) und Interleukin-13 (IL-13). Eine weitere T_{H} Zell-Population, die das Zytokin IL-17 produzieren, sogennante T_{H}17 Zellen, besitzen einen ähnlichen entzündlichen Phänotyp wie T_{H}1 Zellen. IFN-γ-produzierende T_{H}1-Zellen kontrollieren intrazelluläre Pathogene, können jedoch, so wie T_{H}17 Zellen auch, organspezifische Autoimmunkrankheiten, wie beispielsweise Autoimmundiabetes, multiple Sklerose oder Psoriasis auslösen, wohingegen IL-4 produzierende T_{H}2-Zellen schwere organspezifische Autoimmunkrankheiten verbessern können.

Die Differenzierung von nativen CD4⁺-Zellen in entweder T_{H}1- oder T_{H}2-Zellen wird durch Cytokine reguliert, insbesondere durch IL-12 oder IL-4, ferner durch T-Zell-Rezeptor-Ligand-Interaktionen, akzessorische Moleküle und weitere. Außerdem spielt der funktionelle Phänotyp von dendritischen Zellen (DC) eine Rolle, welche entweder T_{H}1-induzierend DC1 oder T_{H}2-induzierende DC2 darstellen. Für die Entstehung von T_{H}17 Zellen ist das Zytokin IL-23 notwendig, dass neben seiner p19 Kette die gleiche p40 Untereinheit wie auch IL-12 besitzt. IL-12 wird, wie IL-23 auch von DC produziert.

Glutathion (GSH) ist ein ubiquitär vorkommendes Tripeptid, das sich aus den nichtessentiellen Aminosäuren Glutamat, Cystein und Glycin zusammensetzt. Wegen seiner Thiolgruppe (SH-Gruppe) und der γ-Glutamylbindung besitzt das Glutathion eine Fülle von biologischen Funktionen, die Entgiftungsreaktionen, Strukturbildung von Proteinen, Coenzymfunktionen, Reparatur von DNA-Schäden, Beeinflussung des Zellmilieus und damit Beteiligung an Entwicklungs- und Alterungsprozessen (Altern) umfassen. Glutathion ist wohl das am häufigsten vorkommende niedermolekulare Thiol und wird in nahezu allen Zellen mit zum Teil relativ hohen Konzentrationen (etwa 5 mmol/l) gefunden. Biosynthese und Abbau des Glutathions verlaufen über den sog. γ-Glutamylzyklus. Es steht als Sulfhydrylpuffer in der Zelle im Gleichgewicht mit dem Glutathion-Disulfid GSSG, das als Oxidationsprodukt entweder durch katalytische Wirkung einer Selen-haltigen Glutathion-Peroxidase entsteht, wobei im Stoffwechsel anfallendes Wasserstoffperoxid reduziert wird oder in einer Transhydrogenierung gebildet wird. Eine NADPH-abhängige GSSG-Reduktase katalysiert die Rückreaktion zu Glutathion.

In neueren Studien (siehe Utsugi et al., 2003, J. Immunol. 171, 628-625) konnte gezeigt werden, dass Verbindungen, die dazu in der Lage sind, Glutathion-S-Konjugate zu bilden, u.a. in Macrophagen eine Lipopolysaccharid-induzierte Interleukin 12-Produktion unterdrücken.

Trotz der aufgezeigten Möglichkeiten zur Behandlung von Autoimmunkrankheiten besteht immer noch ein großes Interesse, Alternativen zu den bisher eingesetzten Therapieansätzen zu finden, da die aufgezeigten Ansätze oftmals schwerwiegende Nebenwirkungen verursachen oder nur mit großem Aufwand und/oder hohen Kosten durchzuführen sind.

Suthanthiran et al. offenbaren Verfahren zur Bestimmung der Inhibierung der Proliferation von T-Zellen durch BSO, sowie die Diskussion der Verabreichung von BSO zur Immunsuppression bei Organtransplantationen.

Die EP 1 354 601 A1 offenbart die Verabreichung von bspw. N,N'-Diacylcystein bei Organtransplantationen nach Messung des Gehalts an reduziertem Glutathion in Makrophagen und Monozyten eines zu behandelnden Patienten.

Aufgabe der vorliegenden Erfindung ist es daher, Substanzen zur Herstellung eines Arzneimittels zu identifizieren, welches zur Prophylaxe und/oder Bekämpfung von T-Zell vermittelten Autoimmunkrankheiten dient.

Erfindungsgemäß wird diese Aufgabe durch die Bereitstellung eines Verfahrens gemäß den anliegenden Ansprüchen gelöst.

Die der Erfindung zugrunde liegende Aufgabe wird dadurch vollständig gelöst.

Die Erfinder konnten aus eigenen Versuchen zeigen, dass durch die Absenkung des zellulären GSH-Gehalts mit Vertretern der genannten Substanzen DC der Gehalt von reaktiven Oxygen Spezies (ROS) ansteigt und gleichzeitig die Produktion von IL-12 und IL-23 gehemmt werden kann. Dadurch wird in den DC eine Ausbildung des DC2-Phänotyps ausgelöst und so die Differenzierung von IFN-γ-produzierenden T_{H}1 Zellen und IL-17 produzierenden T_{H}17 Zellen unterdrückt, und stattdessen die Bildung IL-4-produzierender T_{H}2-Zellen gefördert. Diese können im Gegensatz zu dem IFN-γ-produzierenden T_{H}1-Zellen bzw. IL-17-produzierenden T_{H}17 Zellen keine organspezifischen Immunkrankheiten auslösen.

Die Erfinder haben in weiteren Versuchen gezeigt, dass durch den Einsatz von bspw. L-BSO bei Versuchsmäusen, bei welchen eine experimentelle Autoimmunenzephalomyelitis (EAE) ausgelöst wurde, der Verlauf deutlich milder war, und dass sogar einige Mäuse gesund blieben. Im Gegensatz zur Kontrollgruppe überlebten alle Versuchstiere, denen L-BSO verabreicht wurde.

Damit wird durch die vorliegende Erfindung zum ersten Mal gezeigt, dass durch die kontrollierte Absenkung des zellulären GSH-Spiegels zum einen die IL-12- und IL-23 - Produktion von DC und hieraus resultierend die IFN-γ-und die IL-17-Produktion von T-Zellen gesenkt werden kann und dadurch Autoimmunkrankheiten gezielt und erfolgreich behandelt werden können.

In der vorliegenden Erfindung wird auch aufgezeigt, dass zur Behandlung von Autoimmunkrankheiten zum einen Inhibitoren der GSH-Synthese eingesetzt werden können, wie bspw. L-BSO, oder aber Substanzen, die GSH-S-Konjugate bilden - und somit GSH depletieren - und dadurch den zellulären GSH-Spiegel absenken und den zelluläre ROS-Spiegel ansteigen lassen.

Untersuchungen an Patienten mit Asthma haben gezeigt, dass bestimmte GSH-S-Transferasen Genotypen mit der Entwicklung von Asthma, erhöhten IL-4 und IgE Spiegel und verminderter Detoxifikationskapazitäten einhergehen. Eine typische T_{H}2-Erkrankung der Lunge ist somit mit einer verminderten Detoxifikationskapazität assoziiert, die auf GSH basiert.

Peterson et al., Proc. Natl. Acad. Sci. USA, 1998, berichten in der genannten Publikation über Versuche bei drei immunologischen Modellen, bei welchen durch die GSH-Depletion eine Verlagerung vom typischen T_{H}1-Cytokin-Profil zu den T_{H}2-Reaktionsschemata bewirkt werden konnte. Allerdings zeigte diese Arbeitsgruppe keinen Zusammenhang zu Autoimmunkrankheiten.

Die Erfinder konnten ferner zeigen, dass die GSH-Deprivation (durch eine gezielte Verabreichung von Substanzen, die den GSH-Level absenken) die T-Zell-Cytokinproduktion im Menschen auch *in vivo* beeinflusst werden kann.

Bei der hierin offenbarten Verwendung sind die Autoimmunkrankheiten bspw. ausgewählt aus der Gruppe umfassend: Multiple Sklerose, Diabetes mellitus, rheumatoide Arthritis, Psoriasis, Morbus Crohn, Colitis ulcerosa, Zöliakie, Systemischer Lupus Erythematodes, Ekzemkrankheiten, Transplantatabstoßung, Graft-versus-Host Reaktion, granulomatöse Erkrankungen, insbesondere Sarkoidose und Granuloma anulare. Im Gegenzug sollten sich typische T_{H}2-vermittelte Erkrankungen wie atopisches Asthma, allergisches Asthma, atopische oder allergische Rhinitis durch einen Glutathion-induzierenden Ansatz mit pharmazeutischen Substanzen wie N-Acetylcystein bessern.

Insbesondere ist bevorzugt, wenn die Verwendung bei der multiplen Sklerose erfolgt.

Bei der multiplen Sklerose sind in den Entzündungsherden (Plaques) u.a. T-Lymphozyten, B-Lymphozyten und Makrophagen zu finden, was als Hinweis auf eine Autoimmunreaktion gewertet wird. Zur Therapie der multiplen Sklerose wird momentan insbesondere rekombinant hergestelltes Interferon-β eingesetzt, insbesondere beim schubförmigen Krankheitsverlauf.

Der Autoimmunkrankheit Diabetes mellitus Typ 1 liegt eine zellulär vermittelte chronische und irreversible Zerstörung der Insulin produzierenden β-Zellen des Pankreas zugrunde, wobei vermutlich ein auf den β-Zellen präsentiertes Peptid von autoimmunen T-Lymphozyten erkannt wird, die ihrerseits die β-Zellen zerstören.

Bei der rheumatoiden Arthritis handelt es sich um eine Autoimmunkrankheit, bei welcher eine Veränderung von Synovialzellen eine Immunantwort von T-Lymphozyten, B-Lymphozyten und Makrophagen induziert wird, die zur Antikörperbildung gegen Synovialzellen führt. Als Folge werden Hydrolasen, Collagenasen und lysosomale Enzyme freigesetzt, die zur Zerstörung von Gelenkknorpel führen. Eine Therapie erfolgt momentan mit Antiphlogistika, Corticosteroiden und anderen Antirheumatika.

Mit der hierin offenbarten Verwendung wird ein Ansatz aufgezeigt, mit welchem eine neue Alternative zur Behandlung der aufgezählten Krankheiten ermöglicht wird. Die Erfinder konnten nämlich in eigenen Versuchen zeigen, dass in Mäusen bei einer Verabreichung von u.a. L-BSO der Krankheitsverlauf einer experimentellen autoimmunen Enzephalomyelitis (EAE) deutlich verbessert werden konnte. Der verbesserte Krankheitsverlauf - und dies konnten die Erfinder ebenfalls zeigen - steht in unmittelbarem Zusammenhang mit der Absenkung des GSH-Spiegels, dem Anstieg von ROS und der damit verbundenen Differenzierung von T-Zellen in T_{H}2-Zellen.

Hierein offenbart ist weiterhin eine pharmazeutische Zusammensetzung, die zumindest eine der Substanzen L-BSO oder eine GSH-depletierende Substanz umfasst, insbesondere Ifosfamid, Diethylmaleat oder Acetaminophen, zusammen mit physiologisch verträglichen Hilfsstoffen und/oder Trägersubstanzen enthält.

Solche Inhaltsstoffe sind z.B. Verdünnungsmittel, Bindemittel, Suspensionsmittel, Schmiermittel, Stabilisatoren usw. Dazu zählen, sind aber nicht hierauf beschränkt, z.B. Wasser, Salzlösungen, Alkohole, pflanzliche Öle, Polyethylenglycole, Monoglyceride, etc. Eine Übersicht über derartige Inhaltsstoffe finden sich beispielsweise in A. Kibbe: "Handbook of Pharmaceutical Excipients", 3. Aufl. 2000, American Pharmaceutical Association and Pharmaceutical Press.

Je nach Verabreichung der pharmazeutischen Zusammensetzung, also oral, parenteral, bukkal, nasal, transdermal oder intradermal, wird die Zusammensetzung entsprechend formuliert sein, also beispielsweise in Form von Kapseln, Depot-Kapseln, Tabletten, als flüssige Zubereitungen, die injiziert werden, oder aber als Formulierungen, die über Pumpen verabreicht werden. Die Verabreichung kann demnach auch subkutan oder intravenös erfolgen. Die Verabreichung kann ferner in Abhängigkeit vom Patienten und der Erkrankung, systemisch oder lokal und subtil gerichtet vorgenommen werden. In der pharmazeutischen Zusammensetzung kann darüber hinaus eine Kombination von zwei oder mehreren Substanzen, die den GSH-Level absenken, eingesetzt werden.

Die Verwendung kann in einer Dosis von ca. 0,001 mg/kg Körpergewicht eines Patienten bis ca. 100 mg/kg Körpergewicht eines Patienten erfolgen.

Die Erfindung betrifft wie einleitend erwähnt ein Verfahren zur Bestimmung des Potentials von pharmazeutischen Substanzen, den Glutathiongehalt von Zellen zu senken, mit den folgenden Schritten:
a) Inkubation von Zellen mit unterschiedlichen Konzentrationen der jeweils zu prüfenden pharmazeutischen Substanz;
b1) Bestimmung des Glutathiongehalts der Zellen im Vergleich zu Kontrollzellen, und
b2) Färben der Zellen mit zumindest einem Fluoreszenz- oder Chemilumineszenzfarbstoff, insbesondere mit 2',7' Dichlorofluorescein (H₂ DCFDA), zur Bestimmung des Potentials, ROS zu induzieren,
d) Auswählen der pharmazeutischen Substanzen, die den zellulären Glutathiongehalt senken bzw. den ROS-Gehalt steigern, für einen Einsatz in der Therapie von Autoimmunkrankheiten.

Dabei ist bevorzugt, wenn das Verfahren *in vitro* oder *ex-vivo* durchgeführt wird.

Dabei bedeutet eine "*in-vitro*"-Durchführung des erfindungsgemäßen Verfahrens zum Prüfen des Potentials von pharmazeutischen Substanzen, den Glutathiongehalt von Zellen zu senken, dass Zellen, bspw. Antigenpräsentierende Zellen oder Dendritische Zellen, *in vitro* mit unterschiedlichen Konzentrationen der jeweils zu prüfenden pharmazeutischen Substanz inkubiert werden. Nach einer bestimmten Inkubationszeit, vorzugsweise nach 1 Stunde, 2 Stunden, 24 Stunden oder 48 Stunden wird dann der Glutathiongehalt der Zellen im Vergleich zu Kontrollezellen bestimmt.

Für die Bestimmung des Glutathiongehalts werden die Zellen nach der Inkubationszeit lysiert und auf Eis inkubiert. Das Lysat wird anschließend hochtourig zentrifugiert und der Überstand für die GSH-Messung verwendet, das Sediment für die Proteinmessung (bspw. nach Lowry) aufbereitet. Dabei ist bevorzugt, wenn die Zellen mit Sulfosalicylsäure (SSA), vorzugsweise von 1 % Sulfosalicylsäure, lysiert werden.

Die so aufbereiteten Proben und ein GSH-Standard werden anschließend in einer Mikrotiterplatte verdünnt und die Assay-Reaktion durch Zugabe von einem Reaktionsmix, der Glutathionreduktase enthält, gestartet. Anschließend wird der Anstieg der Extinktion bei 405 nm in 30 s Intervallen über 10 min mit einem Mikrotiterplattenleser gemessen, und der Glutathiongehalt unter Benutzung einer Kalibrierungskurve ermittelt.

Dabei ist bevorzugt, dass, wenn der Glutathiondisulfid- (GSSG-) Gehalt der Zellen, also die oxidierte Form von Glutathion gemessen wird, das GSH, also die reduzierte Form, in der Probe mit bspw. 2-Vinylpyridin bei einem pH zwischen 5 und 7 maskiert wird. Zur Messung des Proteingehaltes jeder Probe wird das Zellpellet nach der Lyse getrocknet. Die Bestimmung des Proteingehalts erfolgt kann vorzugsweise nach der Methode nach Lowry erfolgen (siehe bspw. Dringen et al., "Supply by Astrocytes of CysGly as Precursor for neuronal Glutathione", J. Neurosci 19:562-569).

Zur Durchführung des erfindungsgemäßen Verfahrens *ex-vivo* werden die zu prüfenden pharmazeutischen Substanzen den gewünschten Individuen (Säugetiere) verabreicht. Nach der Behandlung werden dann aus den jeweiligen Organen kleine Gewebestücke entnommen und die in diesen Geweben/Organen enthaltene Zellen aufbereitet und entsprechend untersucht, d.h. die weitere GSH- und Proteinbestimmung erfolgt entsprechend dem Vorgehen wie für das *in vitro*-Verfahren beschrieben.

Der Vergleich zum GSH-Gehalt von Kontrollzellen bzw. Kontrollgewebe zeigt in einem letzten Schritt jeweils die GSH-senkende Wirkung der zu prüfenden pharmazeutischen Substanz.

Mit dem erfindungsgemäßen Verfahren werden reaktive Sauerstoffspezies (ROS) gemessen. Hierzu wird vorzugsweise der Fluoreszenzfarbstoff 2',7' Dichlorofluorescein-Diacetat (H₂ DCFDA) verwendet. Das Absorptionsspektrum dieser Substanz beträgt 492 - 495nm und das Emissonsspektrum liegt im Bereich zwischen 517 - 527nm. Bei Induktion von Zellstress z.B. durch die Gabe von ROS verursachenden Reagenzien, kommt es zu einer Oxidation des H₂DCFDA hin zu DCFDA+H₂. Die dabei entstehende Fluoreszenz kann mittels Durchflusszytometrie detektiert werden (FL1).

Alternativ kann bspw. eine Lucigenin-verstärkte Chemilumineszenz oder eine Coelenterazin-verstärkte Chemilumineszenz eingesetzt werden. Für Fluorimetrische Assays ist neben 2',7' Dichlorofluorescein-Diacetat (H₂ DCFDA) auch Dihydroethidium geeignet. Ferner können auch photometrische Verfahren eingesetzt werden, die bspw. über die Cytochrom C-Reduktion oder die NBT-Reduktion detektierbar sind. Alternativ sind auch Elektronen Spin Resonanz und Spin Trapping als geeignete Nachweisverfahren zu nennen.

Die erfindungsgemäßen Verfahren werden auch als "Drug-Screening"-Verfahren bezeichnet und bieten die Möglichkeit, Substanzen gezielt hinsichtlich ihrer Fähigkeit auszuwählen, den Glutathiongehalt von Zellen zu senken, bzw. den ROS-Gehalt zu steigern. Die in diesem Sinne "positiv" gescreenten Substanzen können dann im Rahmen einer Therapie der weiter oben aufgeführten Krankheiten eingesetzt werden. Das erfindungsgemäße Verfahren stellt somit eine Methode zur Identifizierung neuer Medikamente dar, die entweder zur Therapie der weiter oben genannten Krankheiten dienen können, oder zur Identifizierung von Anti-allergischen/antisklerotischen wirksamen Medikamenten, mit welchen T_{H}2 Erkrankungen wie allergisches/atopisches Asthma und allergische/atopische Rhinitis oder IgE-vermittelten Erkrankungen (Allergien) oder fibrosierende/sklerosierende Erkrankungen (Sklerodermie, Lungenfibrose, Leberfibrose) mit GSH induzierenden, ROS-senkenden pharmazeutischen Substanzen wie N-Acetylcystein behandelt werden können.

Vorliegend wird ferner ein Verfahren zu Bestimmung der genetischen Suszeptibilität, eine Autoimmunkrankheit zu entwickeln, offenbart, mit den folgenden Schritten:
a) Gewinnung von peripheren Blutleukozyten;
b) Isolierung der genomischen DNA aus den peripheren Blutleukozyten;
c) Ermittlung der Genotypen der Glutathion-S-Transferasen (GST) mittels Real-Time-PCR und/oder Gen-Chips.

Mit diesem Verfahren kann auf einfache Weise die Neigung von Individuen, eine Autoimmunkrankheit zu entwickeln, über die Ermittlung der Glutathion-S-Trasnferasen bestimmt werden.

Hierzu werden bei dem Verfahren periphere Blutleukozyten gewonnen und hieraus genomische DNA isoliert. Mittels real-time PCR werden die Genotypen der Glutathion-S-Transferasen (GST) ermittelt. Dadurch können Polymorphismen aufgeklärt werden. Die entsprechenden Primersequenzen für die GST-Familien sind bekannt (siehe bspw. McIlwain et al., "Glutathion S-transferase polymorphisms: cancer incidence and therapy", Oncogene (2006) 1639-1648).

Der Metabolismus und die Detoxifikation von reaktiven Oxygen Intermediaten (ROI) ist teilweise abhängig von den Mitgliedern der GST Familie, insbesondere GSTM1, GSTT1, und GSTP1. Menschen, die homozygot für GSTM1 oder GSTT1 null Gene sind, können theoretisch langsamer ROI beseitigen und sind so einem höheren Risiko von intrazellluärem Zellschaden durch ROS ausgesetzt. Neue Untersuchungen haben gezeigt, dass GSTM1 and GSTP1 adjuvante Effekte bei der Entstehung von allergischem Asthma besitzen. Die PCR-basierte Ermittlung der GST-Genotypen kann daher eine genetische Suszeptibilität für die Entwicklung von Autoimmunerkrankungen ermitteln und gleichzeitig auch Hinweise für den sinnvollen Einsatz Glutathiondepletierender Substanzen geben.

Neben der PCR kann auch eine weitere Screening Methode eingesetzt werden, bspw. die Affymetrix GeneChip® Technologie. Durch die Einführung der Human Exon Array Technologie können nun auch hochauflösende Analysen durchgeführt werden, die auch das Splicen von Genen bzw. Exon Cluster berücksichtigen. Solche hochauflösenden Gene Arrays ermöglichen es, die Gene für die GST bei Patienten genau zu ermitteln, und so Suszeptibilität für Autoimmunerkrankungen oder Therapie mit Glutathion-depletierenden Substanzen hinweisend zu bestimmen.

Dieses Genetische Screening von Patienten hinsichtlich der GST Genotypen und die funktionelle Analyse der GSH/GGSG-Modulation dient der prätherapeutischen Bestimmung der Suszeptibilität für ein therapeutisches Ansprechen auf GSH-depletierende pharmazeutische Substanzen. Dies ist von besonderem Interesse bzw. Wichtigkeit, da beim Menschen aufgrund verschiedener Genotypen der Glutathion-S-Transferasen eine unterschiedliche Kapazität vorliegt, ROS zu beseitigen bzw. den zellulären Glutathion-Gehalt auszufüllen. So kann vor Einleitung einer Therapie mit den genannten Substanzen das mögliche therapeutische Ansprechen vorher eingestuft werden.

Weitere Vorteile ergeben sich aus den Figuren und dem nachfolgenden Beispiel.

Ausführungsbeispiele der Erfindung werden in den nachfolgenden Figuren und in den Beispielen näher erläutert. Es zeigen:
- Fig. 1A: Flusszytometrische Analysen (Dichlorofluorescein): Induktion von ROS in BMDC durch Glutathion Depletion mit DMF oder H₂O₂ (100µM oder 250µM), sowie die Aufhebung der ROS Produktion durch exogene Zugabe von Glutathion GSH-OEt bzw. dem Glutathion-Induktor N-Acetylcystein (NAC).
- Fig. 1B: Flusszytometrische Analysen (Dichlorofluorescein): Die Induktion von ROS in BMDC durch DMF oder L-BSO.
- Fig. 1C: Bestimmung von IL-12 und IL-23 in Kulturüberständen mittels ELISA: ROS wie H₂O₂ senken die Produktion von IL-12. Mit DMF behandelte DC produzieren deutlich weniger IL-12 und IL-23 im Vergleich zu Kontrollen (DMSO) nach Stimulation mit LPS.
- Fig. 1D: Colorimetrische Bestimmung des GSH-Gehalts und ELISA-Bestimmung des IL-12-Gehalts: DMF, aber auch MMF und L-BSO führen zu einer deutlichen Abnahme des intrazellulären GSH Gehalts und damit verbunden zu einer deutlich verminderten Kapazität IL-12 zu produzieren.
- Fig. 2A: Bestimmung von IL-10, IL-12 und IL-23 in Kulturüberständen mittels ELISA: Der intrazelluläre GSH-Gehalt bestimmt den Phänotyp der DC. DC^{GSHhoch} produzieren IL-12 und IL-23 und zeigen einen DC1 Phänotyp, wohingegen DC2^{GSHniedrig} zwar IL-10 produzieren, aber kaum IL-12 und IL-23. Der Glutathion-depletierende und DC2-induzierende Effekt von DMF kann durch die gleichzeitige exogene Gabe von GSH vollständig aufgehoben werden. Die mit GSH wieder aufgefüllten DC zeigen einen DC1 Phänotyp
- Fig. 2B/C: Bestimmung von IFN-γ und IL-4 mittels ELISA: Die intrazellulären GSH-Level bestimmen den funktionellen Phänotyp IL-12⁺DC1^{GSHhoch} oder IL-12-DC2^{GSHniedrig} und so die Differenzierung von TCR-transgenen T Zellen in Richtung IFN-γ produzierender Th1 Zellen oder IL-4 produzierender Th2 Zellen.
- Fig.2D: Bestimmung von IL-17 mittels ELISA: In gleicher Weise bestimmen die intrazellulären GSH-Level den funktionellen Phänotyp von IL-23+DC^{GSHhoch} oder IL-23 DC2^{GSHniedrig} und somit die Generierung von Th-17⁺ Zellen oder Th-17 Zellen. Die FACS Abbildungen rechts zeigen oben die Kontrolle, unten DMF.
- Fig. 3A/B: Bestimmung von IFN-γ und IL-4 mittels ELISA: In *vitro*-Stimulation von PLP-spezifischen T-Zellen nach GSH-Deprivation der DC fördert ähnlich wie IL-4 die Differenzierung in Richtung Th2 und hemmt die Differenzierung von T_{H}1 und T_{H}-17.
- Fig. 3C: Verlauf der EAE mit und ohne Behandlung: *In vitro,* unter GSH-Deprivation generierte, autoreaktive T-Zellen sind, ähnlich wie *in vitro,* in Anwesenheit von IL-4, generierte T_{H}2 Zellen, kaum enzephalitogen.
- Fig. 4A: Verlauf der EAE mit und ohne Behandlung: Die *In-vivo*-Depletion von GSH durch DMF (oben) oder L-BSO (unten) schützt aktiv immunisierte Mäuse vor dem Ausbruch einer schweren Enzephalomyelitis.
- Fig. 4B: Bestimmung von IL-4 und IFN-γ: Die *In-vivo*-Depletion von GSH fördert die Differenzierung von autoreaktiven T_{H}2 Zellen.
- Fig. 5A: Bestimmung von GSH: *In-vitro*-Depletion von GSH in menschlichen DC durch DMF.
- Fig. 5B: PASI Verlauf von 9 Patienten, die mit Fumaderm® behandelt wurden.
- Fig. 5C: Ergebnisse der Cytokinanalysen peripherer Lymphozyten aus Blut von Patienten, bei welchen durch eine GSH-senkende Therapie T_{H}2-Antworten induziert wurden.

### Beispiel I

### 1. Material und Methoden

### 1.1 Versuchstiere

Weibliche SJL Mäuse, C57BL/6, OVA transgene DO11.10-BALB/c Mäuse und BALB/c Mäuse wurden entweder aufgezüchtet oder gekauft (Charles River Laboratories, Harlan) und unter spezifischen pathogenfreien Bedingungen gehalten. Die Tierversuche waren behördlich genehmigt.

### 1.2

Zur Messung reaktiver Sauerstoffspezies (ROS) in DC und APC wurde der Fluoreszenzfarbstoff 2',7' Dichlorofluorescein (H₂ DCFDA, Molecular Probes, Eugene, USA) verwendet. 5 x 10⁵ DC wurden in 1ml RPMI-Medium (ohne Mercaptoethanol) 60 min bei 37°C im Brutschrank mit 1 % DMSO oder 10 µg/ml DMF +/- NAC(1mM) oder GSX(1mM) vorinkubiert. Nach dieser Vorinkubation wurde 1µM/ml H₂DCFDA zugegeben und die Zellen für weitere 30min bei 37°C inkubiert. Innerhalb einer Stunde wurde die entstandene Fluoreszenz mittels Durchflusszytometrie detektiert (FL1).

### 1.3 Glutathionbestimmung

Zelluläres GSH und GSSG wurde in Mikrotiterplatten-Assays mittels colorimetrischer Verfahren bestimmt. Der zelluläre GHS-Gehalt wird als Prozent des GSH-Levels in frisch präparierten Kontrollen bestimmt oder als pmol 10⁻⁶ Zellen. GSSG war nicht detektierbar.

### 1.4 Cytokinassays

IL-4, IL-10, IL-12 und Interferon-γ wurden mittels des ELISA (enzyme linked immunosorbent assay)-Sets oder unter Verwendung einzelner Komponenten von BD BioSciences bestimmt. IL-23 im Überstand wurde mit Hilfe des ELISA Sets (eBioscience) bestimmt. Für intrazytoplasmatische Zytokinanalysen wurden die T Zellen mit PMA/Ionomycin oder mit APC und Peptid restimuliert in Anwesenheit von Brefeldin A, im Anschluss fixiert und permeabilisiert und gefärbt. Für die Oberflächenfärbung wurden anti-CD4 oder KJ1-26 Antikörper verwendet, die Zytokin-Antikörper (PE markiert) stammten alle von BD Pharmingen.

### 1.5 Antigen präsentierende Zellen

Als Antigen präsentierende Zellen (APC) wurden T-zell depletierte Milzzellen von syngenen Mäusen verwendet. Dendritische Zellen (DC), die aus Knochenmark gewonnen wurden (BMDC) und mit GM-CSF kultiviert wurden, wurden am Tag 7 der Kultur für die jeweiligen Versuche verwendet. Für den Versuch wurden jeweils eine gleiche Anzahl an APC entweder mit dem Medium alleine, mit L-BSO (C₈H₁₈N₂O₃S, 0,5 mM), Dimethylsulfoxid DMSO (C₂H₆OS), Dimethylfumarat DMF (C₆H₈O₄, gelöst in DMSO, 70 µM), oder Calcium-Monomethylfumarat (MMF; CaC₁₀H₁₀O₈, 200 µM) kultiviert. Alle Substanzen bis auf MMF (Fumapharm) wurden von Sigma-Aldrich bezogen. Zu den jeweils angegebenen Zeiten (siehe unten) wurden die Zellen entweder direkt hinsichtlich ihres GSH-Gehalts analysiert, zur T-Zell-Stimulierung verwendet oder mit *Escherichia coli* Lipopolysacchariden 055.B5 (Sigma-Aldrich) oder CpG-DNA 1668 (MWG Biotech) und IL-4 (Stratmann) zur IL-2-Produktion stimuliert. Um den GSH-Level wieder anzuheben, wurde 1 mM GSH-OEt (C₁₂H₂₁N₃O₆S; Sigma-Aldrich) oder 1mM NAC (Sigma-Aldrich) hinzugefügt. Die GSH-Speicher in Mäusen wurden durch Fütterung mit DMF oder MMF depeltiert, in Menschen durch Behandlung mit Fumaderm.

### 1.6 T-Zellen und T-Zellstimulation

Die CD4⁺-T-Zellen von DO11.10 Mäusen wurden mittels MACS-Magnetbeads (Milteny Biotech) sortiert. Durch eine Kultivierung von DC an Tag sieben mit entweder Medium und DMSO oder DMF für 2 Stunden konnten DC^{GSHhoch} oder DC^{GSHniedrig} generiert werden. Nach einer vierstündigen Stimulation mit LPS oder CpG-DNA/IL-4 wurden die DC^{GSHhoch} oder DC^{GSHniedrig} zur Stimulierung von DO11.10 T-Zellen mit 10 µg/ml Ovalbuminpeptid 323-339 über zwei Tage eingesetzt. Die T-Zellen wurden mit 50 U/ml IL-2 (Chiron Therapeutics) expandiert. Die Zellen wurden am Tag 10 gewaschen und mit frischem APC restimuliert. Die Analyse erfolgte mit den Überständen nach 24 h. Aus mit PLP-Peptid immunisierten SJL-Mäusen wurden CD4⁺-T-Zellen mittels MACS-Magnetbeads gewonnen und mit bestrahlten APC und PLP Peptid 139-151 stimuliert in Anwesenheit von L-BSO, DMF oder nur in Medium als Kontrolle.

### 1.7 PLP-spezifische T-Zellen und EAE

Milz- und Lymphknoten-CD4⁺ T-Zellen wurden mit PLP (Proteolipid Protein) Peptid 139-151 (10 µg/ml) stimuliert und bestrahlten SJL-APC in Gegenwart oder Abwesenheit von L-BSO (0,5mM, Sigma) vier Tage lang stimuliert, für drei bis vier Tage expandiert und anschließend zur Cytokinproduktion restimuliert. (Das Proteolipid-Protein ist das häufigste Myelinprotein im zentralen Nervensystem und spielt als Autoantigen eine wichtige Rolle bei der Entstehung der Multiplen Sklerose). Anschließend wurden 10⁷ sham- (Kontrolle) oder L-BSO-behandelte T-Zellen intraperitoneal in naive SJL Mäuse injiziert und die sich dadurch entwickelnde EAE beobachtet. Zur Behandlung von aktiv induzierter EAE wurden SJL-Mäuse mit 75ng in 4mg/ml Complete Freund's Adjuvanz (CFA) immunisiert, anschließend 200 µg Pertussistoxin intraperitoneal injiziert und die Mäuse bezüglich der Entwicklung der EAE beobachtet. Die Hälfte der Gruppe wurde mit DMF 0,3mg/ml (Fluka) oder L-BSO 0,15mg/ml im Trinkwasser 14 Tage lang täglich gefüttert. Zur Cytokinanalyse von PLP-spezifischen T Zelllen wurden die drainierenden Lymphknoten der SJL-Mäuse 6 Tage nach der Immunisierung entnommen und CD4⁺ T Zellen Antigenspezifisch analyseirt (ELISPOT). Hierzu wurden ELISPOT Platten (Millipore Corporation) über Nacht bei 4°C mit anti-IFN-γ oder anti-IL-4 Antikörper beschichtet, nach 12h mit PBS/10% FCS geblockt und nach Abkippen der Lösung wurden die isolierten T-Lymphozyten mit APC und Medium allein oder in Anwesenheit von PLP Peptid inkubiert. Nach der Inkubationszeit wurden die Zellen abgekippt, die Platten mit PBS/Tween gewaschen und mit biotinkonjugierten Zweitantikörpern inkubiert. Nach erneuten Waschgängen und Inkubation mit Streptavidin-Alkalische Phosphatase (Boehringer) wurden die Platten mit Entwicklungslösung inkubiert. Nach Waschen mit H2O wurden die Anzahl der Spots im ELISPOT Reader (Biosys) bestimmt.

### 1.8

DC aus menschlichem Knochenmark (BMDC) (CD14⁺) wurden durch MACS (Miltenyi Biotech) isoliert und sieben Tage lang mit IL-4 (500 U/ml, R & D Systems) und GM-CSF (Granulocyten-Monocyten-Kolonie stimulierender Faktor) (1000 U/ml (Immunonex)) inkubiert. GSX Bestimmung wie unter 1.3.

### 1.9 Psoriasis-Therapie und klinische Evaluation

Patienten mit schwerer Psoriasis wurden mit Fumaderm® (Hermal) therapiert. Die Schwere der Krankheit der Patienten wurde unter Verwendung des "Psoriasis Area and Severity Index" (PASI) bestimmt.

### 1.10 Ex-vivo-Cytokinanalyse von menschlichen T Zellen

Das ex *vivo*-Cytokinmuster der T-Zellen wurde zu den angegebenen Zeiten (siehe unten) bestimmt, und zwar jedesmal dann, wenn bei den Fumaderm-Patienten die Routineblutanalyse durchgeführt wurde. PBMC wurden aus heparinisiertem Blut durch Fikollgradientenzentrifugation isoliert und direkt mit Phorbolmyristat-Acetat (PMA); 5 ng/ml) und Ionomycin (0,5 µg/ml) in Gegenwart von Brefeldin A (3 µg/ml) (jeweils von Sigma) vier Stunden lang stimuliert. Anschließend wurde eine Oberflächenfärbung mit Fluoreszin-Thiocyanat(FITC)-konjugierten monoklonalen Antikörpern - entweder gegen CD4 oder CD8 - und eine intrazelluläre Färbung mit Phycoerythrin(PE)-konjugiertem monoklonalen Antikörpern gegen IL-4 oder IFN-γ (BD PharMingen) gemäß dem Protokoll der Hersteller durchgeführt. Ferner wurden Isotypenkontrollen für die Festsetzung der Fenster durchgeführt. Die Proben wurden mit einem FACScan (BD BioSciences) analysiert. Für die in *vitro*-Untersuchungen der T-Zellen wurden PBMC mit 100 ng/ml Staphylokokkenenterotoxin B (SEB) in Gegenwart oder Abwesenheit von DMF (Sigma-Aldrich) drei Tage lang stimuliert und anschließend die Zellen mit IL-2 expandiert. An Tag 13 wurden die Zellen mit PMA und Ionomycin stimuliert und der intrazelluläre IL-4 oder Interferon-γ-Gehalt wurde durch FACS-Analyse bestimmt.

### 2. Ergebnisse

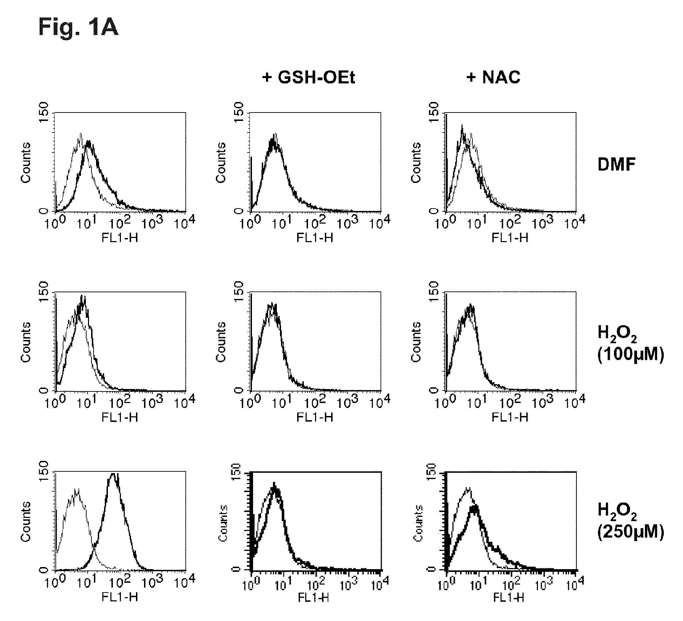

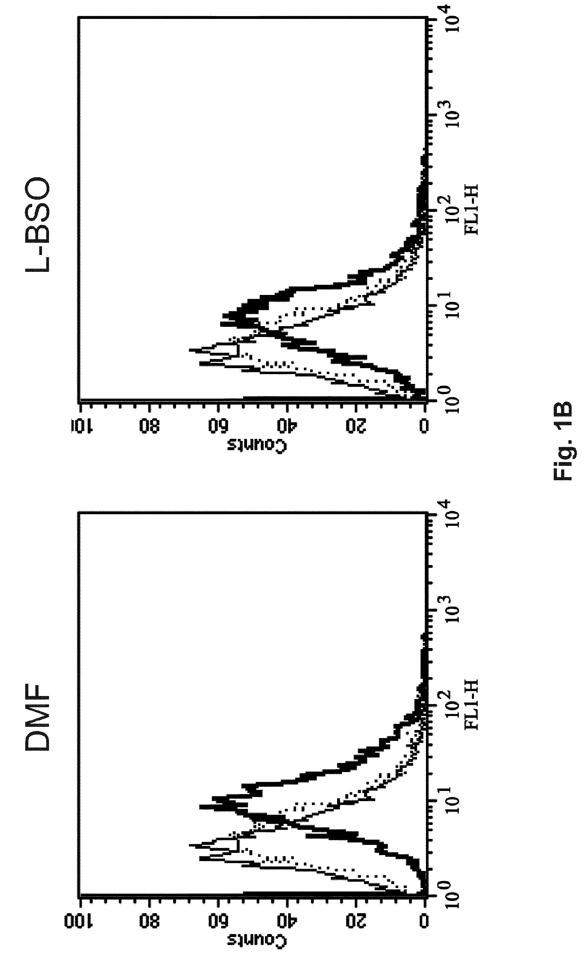

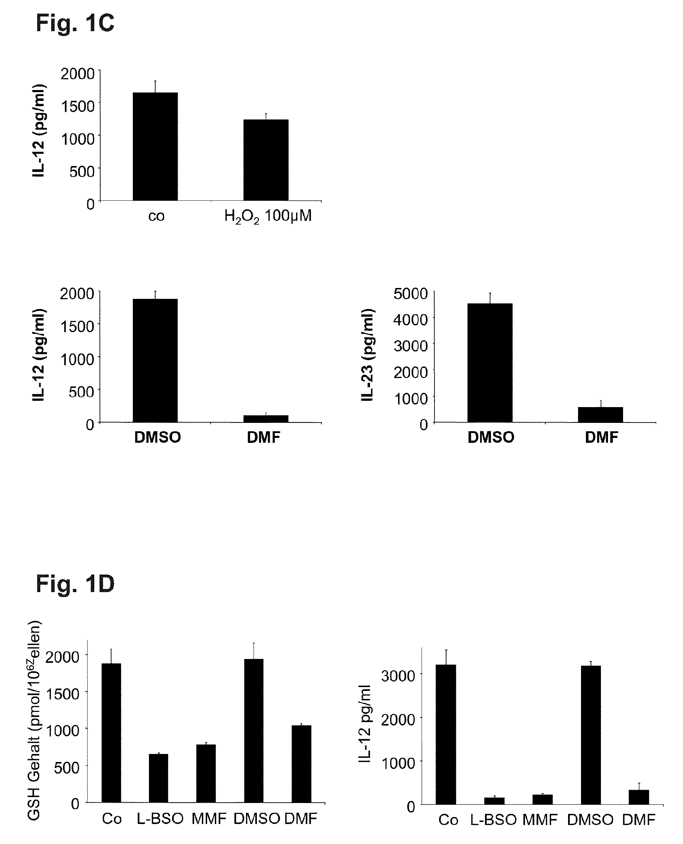

Glutathion ist ein zentrales intrazelluläres Molekül, das an vielen metabolischen Prozessen wie der Detoxifikation von intrazellulären Sauerstoffradikalen (Reactive oxygen species, ROS) beteiligt ist. Oxidativer Stress mit Wasserstoffperoxid (H₂O₂) von DC kann durch Dichlorofluorescein (DCF) sichtbar gemacht werden (Fig. 1A). Glutathion-Depletion durch L-BSO führt zu einem Anstieg von intrazellulären ROS in DC (Fig. 1B). Ebenso kann der Glutathion-S-Konjugatbildner DMF zu einem Anstieg von ROS in DC führen (Fig. 1A+B). Dieser DMF-induzierte ROS Anstieg kann durch Zugabe von GSH Ethylester (GSH-OEt) oder NAC komplett aufgehoben werden (Fig. 1A). Die Zugabe von DMF, MMF oder L-BSO induziert ROS in DC und depletiert gleichzeitig den intrazellulären Glutathionspiegel von DC (Fig.1D). Diese DC zeigen eine starke Suppression der IL-12 Produktion (Fig. 1C+D). Neben IL-12 wird auch die Produktion von IL-23 durch den intrazellulären Glutathion Gehalt bestimmt (Fig.1C). Eine Glutathion-Depletion in DC mit DMF senkt die LPS-induzierte IL-23 Produktion in aus Knochenmark kultivierten DC bis zu 10.000-fach (Fig. 1C). Gleichzeitig induziert DMF einen Anstieg der IL-10 Produktion in DC.

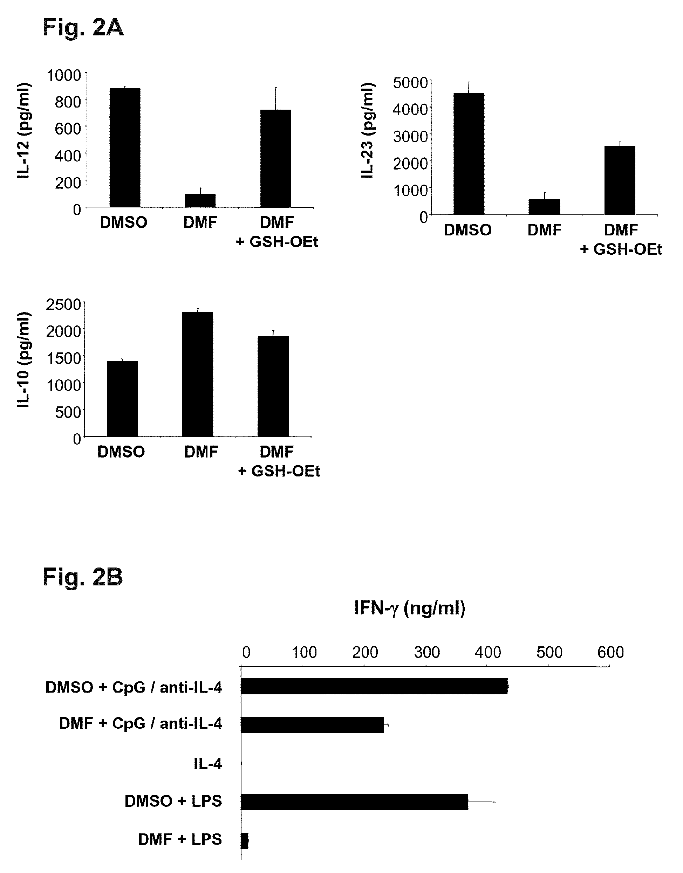

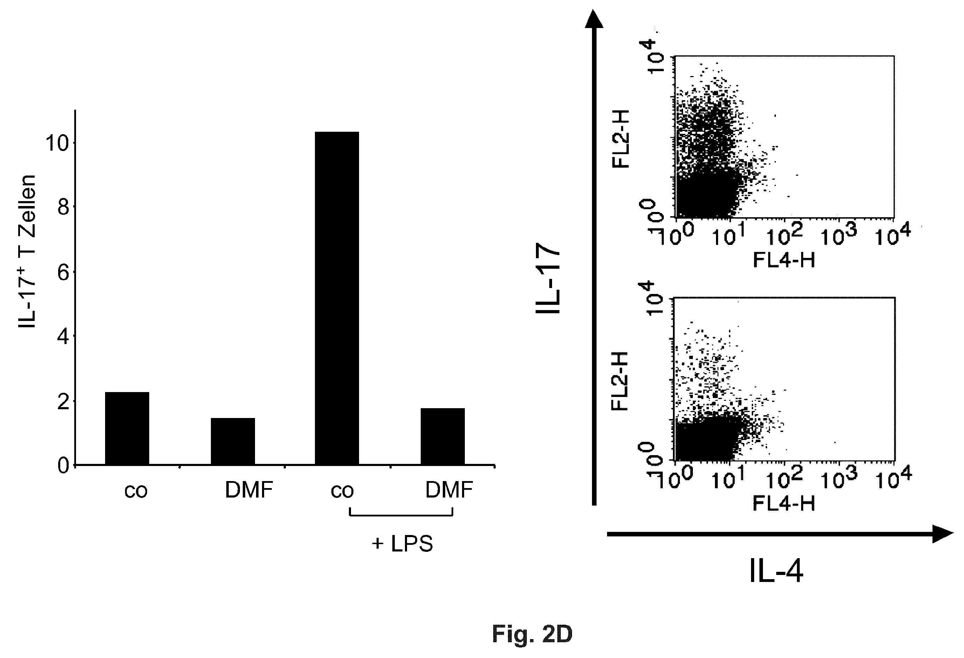

Um auszuschließen, dass die einzelnen Verbindungen irgendwelche pharmakologischen Effekte auf die IL-12-Synthese bewirken, wurde der intrazelluläre GSH-Verlust durch Hinzufügung von exogenem GSH-OEt verhindert. Die Inhibierung der intrazellulären GSH-Deprivation hob wiederum die IL-12-Suppression (Fig. 2A) auf, was beweist, dass die DC einen GSH*^{hoch}*-Status benötigen, um IL-12 zu produzieren. Die Zugabe von NAC zeigte die gleichen Effekte wie GSH-OEt (nicht gezeigt).

Die Zugabe von GSH-OEt zu DMF stellte außerdem die IL-23 Produktion in DC wieder her und erniedrigte die IL-10 Produktion in DC (Fig. 2A). Diese Ergebnisse zeigen, dass der GSH Gehalt den DC Phänotyp und die Zytokinproduktion bestimmt.

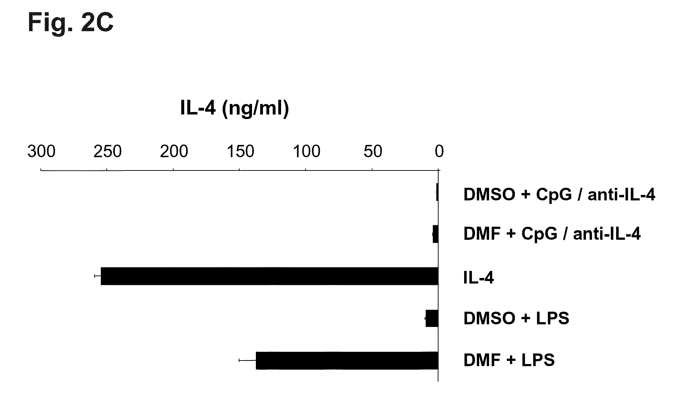

Um zu zeigen, dass der durch DMF veränderte DC Phänotyp in der Lage ist, auch die T Zell Antwort zu beeinflussen wurden Ovalbuminspezifische CD4⁺ T Zellen unter T_{H} (CpG/anti-IL-4 oder LPS) oder T_{H}2 (IL-4) Bedingungen mit DC geprimt und mit DC verglichen, die mit Lösungsmittel, DMF oder DMF und LPS behandelt wurden. Nach Expansion der T_{H} Zellen mit IL-2 über 10-12 Tage wurden die T_{H} Zellen dann mit frischen, irradiierten APC und Antigen restimuliert. IL12⁺ DC^{GSHhoch}, die mit Lösungsmittel oder unter T_{H}1 Bedingungen behandelt wurden, induzieren T_{H}1 Zellen, die viel IFN-γ und kein oder kaum IL-4 produzieren (Fig. 2B). IL-4 oder DMF behandelte DC induzieren dagegen T_{H}2 Zellen, die viel IL-4 und kaum IFN-γ produzieren (Fig. 2C). Sogar unter T_{H}1 Bedingungen (DMF und LPS) induzieren GSH^{niedrig}IL12⁻ DC IL-4 produzierende T_{H}2 Zellen (Fig. 2B). Außerdem induzieren IL23⁻ DC^{GSHniedrig} nach Stimulation mit LPS IL-17⁻T_{H} Zellen, wohingegen IL23⁺ DC^{GSHhoch} IL-17⁺ T_{H} Zellen induzieren.

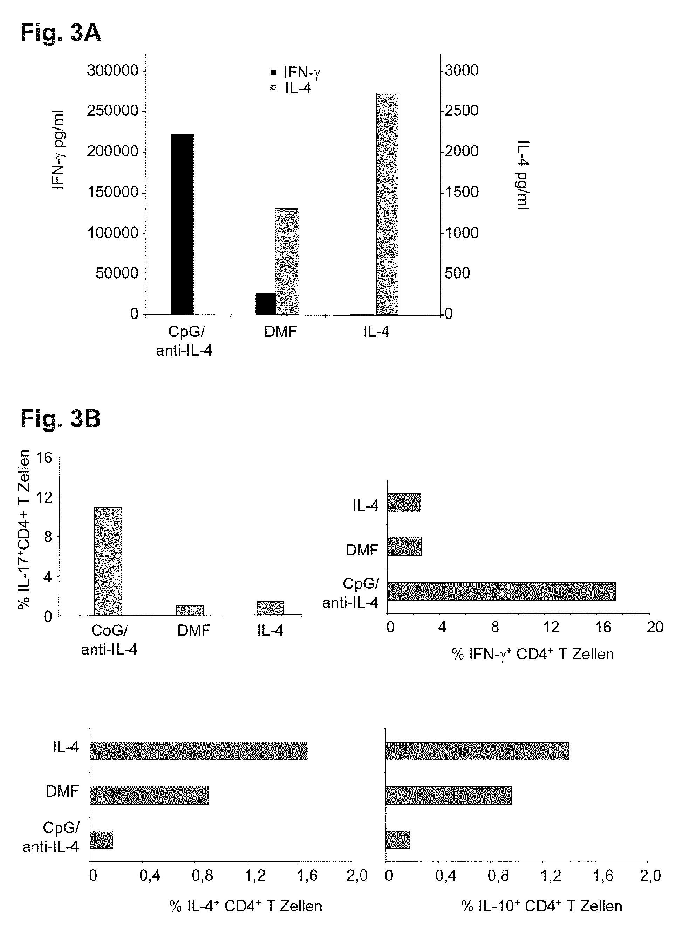

In einem anderen System, in dem T Zellen verwendet wurden, die gegen ein Gehirn-spezifisches Protein gerichtet sind (Proteolipid Protein, PLP) fördert die in *vitro*-Stimulation dieser PLP-spezifischen T-Zellen unter GSH-Deprivation mit DMF ähnlich wie IL-4 die Differenzierung in Richtung Th2 und hemmt die Differenzierung von Th1 und Th-17 (Fig. 3A und B).

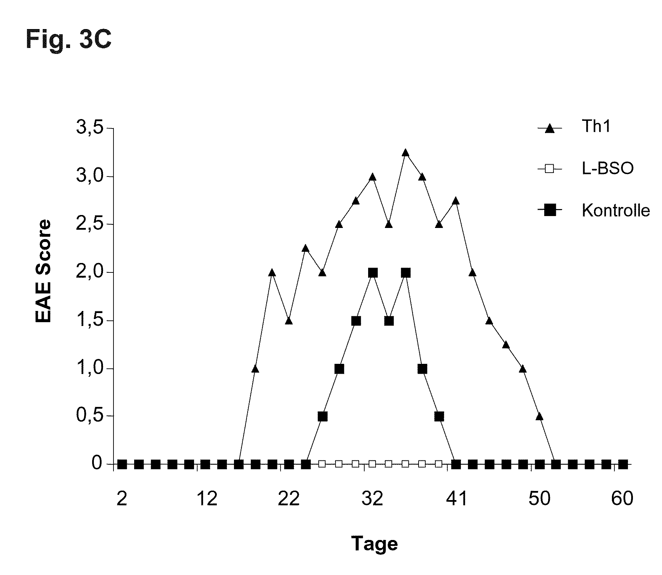

Da die Induktion des T_{H}2-Phänotyps die Fähigkeit der CD4⁺ T-Zellen, Autoimmunkrankheiten in immunkompetenten Mäusen zu induzieren, beeinträchtigen könnte, wurde gefolgert, dass GSH*^{niedrig}*IL12⁻ DC2 die Fähigkeit autoreaktiver CD4⁺-T-Zellen, die Krankheit zu induzieren, abschwächen könnten. Für die nachfolgenden Versuche wurden Lymphozyten aus SJL Mäuse zweimal mit PLP Peptid stimuliert, und zwar entweder in Gegenwart oder Abwesenheit von L-BSO, DMF oder MMF. Nach der zweiten Stimulation wurden 10⁷ Lymphozyten in naive SJL-Mäuse injiziert. Die Ergebnisse dieser Studien sind in Fig. 3C exemplarisch für L-BSO gezeigt. Autoreaktive T Zellen, die mit L-BSO *in vitro* generiert wurden, also unter GSH*^{niedrig}* Bedingungen, waren kaum noch in der Lage eine EAE *in vivo* zu induzieren. Dagegen lösten *in vitro* differenzierte Th1 Zellen oder Th Zellen, die nur mit PLP Peptid und APC ohne weitere Stimulantien aktiviert wurden eine schwere Enzephalomyelitis aus. In Anwesenheit von DMF oder MMF generierte PLP-spezifische T Lymphozyten waren auch nicht in der Lage eine schwere Enzephalomyelitis auszulösen (nicht gezeigt).

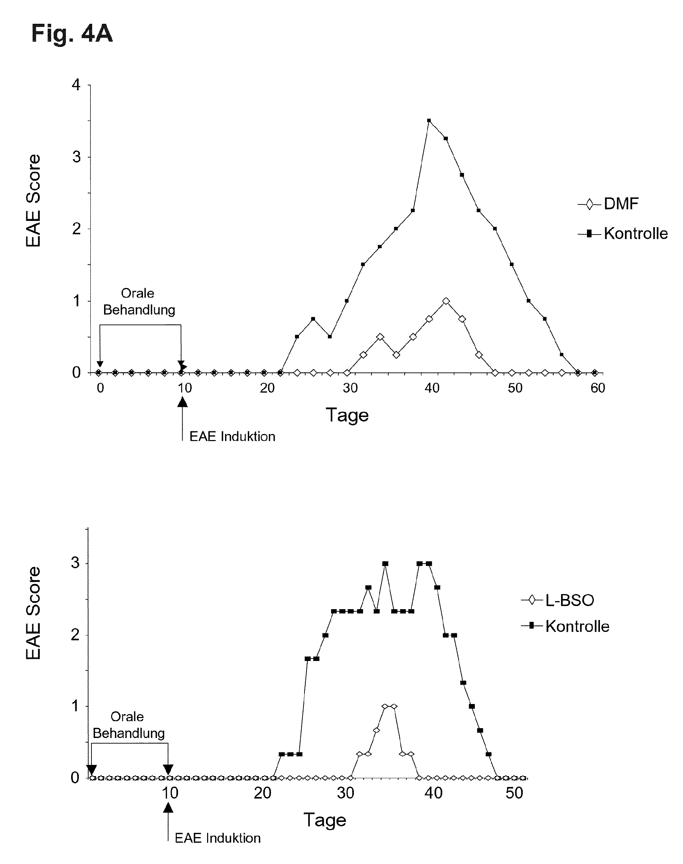

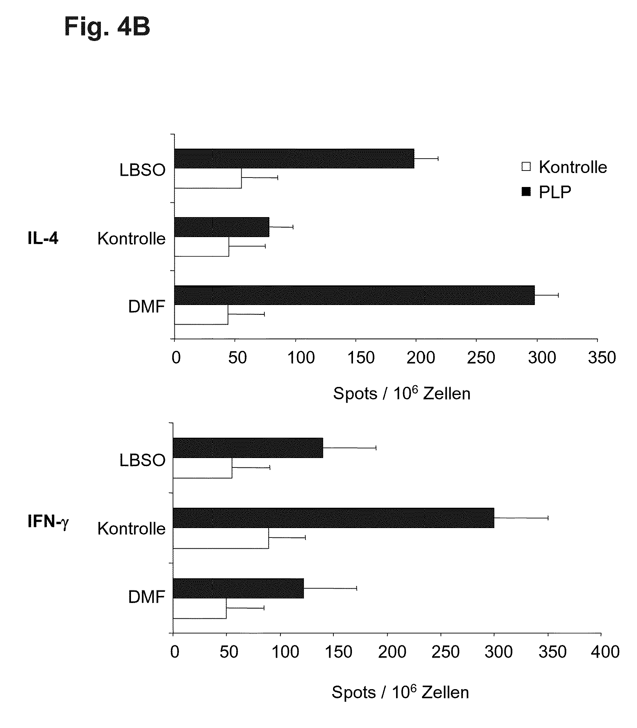

Um ferner zu untersuchen, ob die GSH-Deprivation die Differenzierung und Pathogenizität von autoreaktiven T-Zellen auch *in vivo* beeinflussen kann, wurden Mäuse mit DMF oder L-BSO im Trinkwasser gefüttert, die Kontrollgruppe nur mit Trinkwasser. Eine aktive EAE Induktion erfolgte am Tag 10 mit PLP-Peptid in Complete Freud's Adjuvanz (CFA) und Pertussistoxin. Die Mäuse wurden täglich auf den Verlauf der künstlich ausgelösten Krankheit untersucht. Durch die Verabreichung von L-BSO oder DMF konnten die GSH-Level in lymphoiden Organen und im Hirn von immunisierten Mäusen gesenkt werden (nicht gezeigt). PBS-gefütterte Mäuse entwickelten eine schwere EAE, während L-BSO oder DMFgefütterte Mäuse eine deutlich mildere EAE zeigten, einige Mäuse sogar gesund blieben (Fig. 4A+B) und alle Mäuse überlebten. 6 Tage nach Immunisierung wurden die drainierenden Lymphknoten der Mäuse analysiert. Dabei wurden die CD4⁺ T-Zellen isoliert und die Cytokin-Produktion von PLP-spezifischen T-Zellen mittels ELISPOT nach *in vitro* Stimulation gemessen. CD4⁺-T-Zellen von DMF- und L-BSO gefütterten Mäusen zeigten ein T_{H}2-ähnliches Muster, wohingegen PLP-spezifische CD4⁺-Zellen von PBS-behandelten Mäusen als T_{H}1-Zellen identifiziert wurden (Fig. 4B).

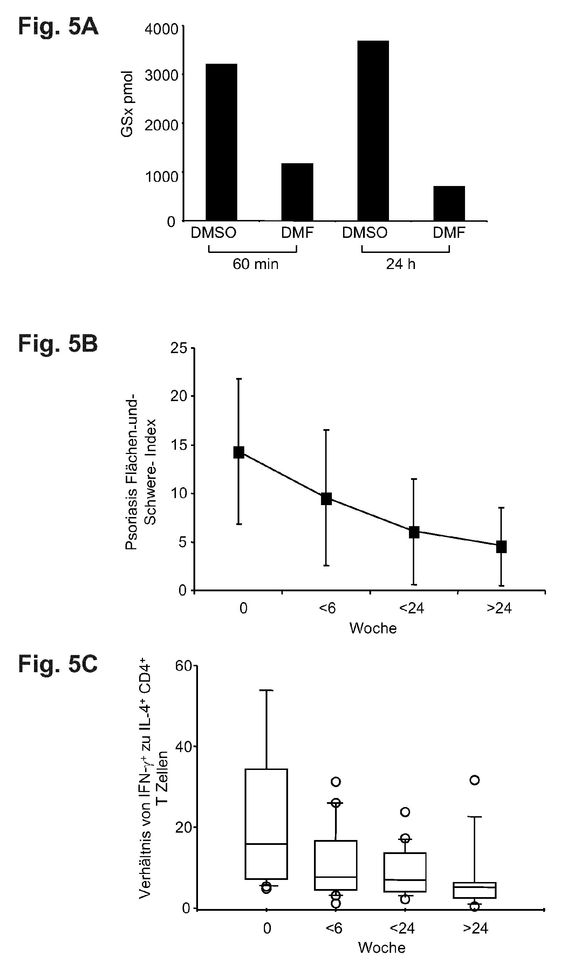

DMF depletiert GSH auch in menschlichen DC *in vitro* (Fig. 5A). Um zu bestimmen, ob die GSH-Deprivation die T-Zell-Cytokinproduktion in Menschen auch *in vivo* beeinflusst, wurde die Interferon γ- und Interleukin 4-Produktion durch CD4⁺- oder CD8⁺-T-Zellen in 18 Patienten bestimmt, die Fumaderm zur Behandlung von Psoriasis erhielten.

Durch die Versuche konnte gezeigt werden, dass eine anhaltende klinische Besserung der Krankheit stark mit zunächst einem Anstieg in der IL-4-Produktion einherging und anschließend mit einem Absenken der IFN-γ-Produktion von CD4⁺-Zellen, was zu einem ständig unterdrückten IFN-γ/IL-4-Verhältnis in der CD4⁺-T-Zellpopulation führte (Fig. **5B****:** Psoriasis Area and Severity Index (PASI) und Fig. 5C: Verhältnis der Interferon-γ-/IL-4⁺-CD4⁺ T-Zellen in PBMC während der Fumaderm®-Therapie).

Durch die von den Erfindern durchgeführten Versuche konnte also gezeigt werden, dass intrazelluläre GSH-Level durch drei vollkommen unterschiedliche Ansätze moduliert werden konnten: Zum einen wurden die GSH-Speicher durch Einsatz von Thiol-depletierenden Agenzien geleert; ferner wurde die GSH-Synthese mit L-BSO inhibiert und außerdem die intrazellulären GSH-Level mit GSH-OEt selektiv wiederhergestellt. Insgesamt konnte durch diese drei Ansätze gezeigt werden, dass durch den intrazellulären GSH-Level die DC-Differenzierung in entweder GSH*^{hoch}*IL-12⁺ DC1 oder GSH^{niedrig}IL-12⁻ DC2 geleitet werden konnte. Diese Regulierung ist - zumindest teilweise - ein essentieller Schritt in der IL12-Produktion. Die Unterschiede in der IL-12-Produktion erklären auch die Eigenschaft von GSH*^{hoch}*DC1 oder GSH*^{niedrig}*DC2 zur Induktion von jeweils T_{H}1 oder T_{H}2.

Mit der vorliegenden Erfindung, also der gezielten Absenkung des zellulären GSH-Level durch bestimmte Substanzen, wird daher ein neuer Ansatz für die Therapie von Autoimmunkrankheiten dargelegt, sowie für Krankheiten allgemein, die sich durch die Absenkung des GSH-Levels behandeln lassen.

### Beispiel II

Zum Überprüfen des Potentials von pharmazeutischen Substanzen, den Glutathiongehalt von Zellen zu senken, wurde folgendes Verfahren durchgeführt: Zellen (Antigenpräsentierende Zellen oder Dendritische Zellen) werden mit unterschiedlichen Konzentrationen der jeweils zu prüfenden pharmazeutischen Substanz inkubiert. Nach einer Inkubationszeit von 1h, 2h, 24h oder 48h wird dann der Glutathiongehalt der Zellen im Vergleich zu Kontrollezellen bestimmt. Die Zellen werden hierzu nach der Inkubationszeit mit 1 % Sulfosalicylsäure (SSA) lysiert und auf Eis inkubiert. Das Lysat wird anschließend hochtourig zentrifugiert und der Überstand für die GSH-Messung verwendet, das Sediment für die Proteinmessung nach Lowry aufbereitet.

Die Proben und der GSH-Standard wurden in einer Mikrotiterplatte verdünnt und die Assay-Reaktion durch Zugabe von einem Reaktionsmix, der Glutathionreduktase enthält, gestartet. Der Reaktionsmix war folgendermaßen zusammengesetzt: 92,7 µl GSH-Puffer (0,1 M Natriumphosphat, Na2HPO4 u NaH2PO4 2H2O, Roth, Karlsruhe, Deutschland), 4 µl 10 mM NADPH (Tetranatriumsalz, AppliChem, Darmstadt, Deutschland), 3 µl 10 mM DTNB (5,5'-Dithiobis (2-Nitrobenzoesäure), Sigma), 0,64 µl Glutathionreduktase (GR, Roche Applied Science, Grenzach-Wyhlen, Deutschland). Der Anstieg der Extinktion wird bei 405 nm in 30 s Intervallen über 10 min mit einem Mikrotiterplattenleser gemessen. Der Glutathiongehalt wurde unter Benutzung einer Kalibrierungskurve ermittelt. Um den Glutathiondisulfid- (GSSG-) Gehalt der Zellen, also die oxidierte Form von Glutathion zu messen, musste das GSH, also die reduzierte Form, in der Probe mit 2-Vinylpyridin bei einem pH zwischen 5 und 7 maskiert werden. Zur Messung des Proteingehaltes jeder Probe wurde das Zellpellet nach der Lyse getrocknet und in 0,5 M NaOH aufgenommen. Die Bestimmung des Proteingehalt erfolgte nach der Methode nach Lowry (siehe bspw. Dringen et al., "Supply by Astrocytes of CysGly as Precursor for neuronal Glutathione", J. Neurosci 19:562-569). Bovines Serumalbumin wurde als Standard benutzt.

Die zu prüfenden pharmazeutischen Substanzen können auch *ex-vivo* auf ihr Potential GSH zu depletieren analysiert werden. Nach Behandlung von Versuchsmäusen können hierzu aus den jeweiligen Organen kleine Gewebestücke entnommen und in etwa 1 ml 1% SSA gelegt werden. Das Gewebestück in SSA wird mit Hilfe einer Ultraschallspitze homogenisiert. Die weitere GSH und Proteinbestimmung erfolgt entsprechend dem Vorgehen wie für APC und DC beschrieben. Der Vergleich zum GSH Gehalt von Kontrollzellen bzw. Kontrollgewebe zeigt die GSH-senkende Wirkung der zu prüfenden pharmazeutischen Substanz.

### Beispiel III

Zur Überprüfung des Potentials von pharmazeutischen Substanzen, den ROS zu induzieren, wurde folgende Verfahren entwickelt: Zur Messung reaktiver Sauerstoffspezies (ROS) bei Zellen wird der Fluoreszenzfarbstoff 2',7' Dichlorofluorescein (H₂ DCFDA) verwendet. Das Absorptionsspektrum der Substanz beträgt 492 - 495nm und das Emissonsspektrum liegt im Bereich zwischen 517 - 527nm. Bei Induktion von Zellstress z.B. durch die Gabe von ROS verursachenden Reagenzien, kommt es zu einer Oxidation des H₂DCFDA hin zu DCFDA+H_{2.} Die dabei entstehende Fluoreszenz kann mittels Durchflusszytometrie detektiert werden (FL1).

## Patentansprüche

1. Verfahren zur Bestimmung des Potentials von pharmazeutischen Substanzen, den zellulären Glutathiongehalt zu senken und den zellulären reaktiven Sauerstoffspezies (ROS) - Gehalt zu steigern, und damit des Potentials der pharmazeutischen Substanzen, zur Therapie von T-Zell vermittelten Autoimmunkrankheiten eingesetzt zu werden, mit den folgenden Schritten:
a) Inkubation von Zellen mit unterschiedlichen Konzentrationen der jeweils zu prüfenden pharmazeutischen Substanz; und
b1) Bestimmung des Glutathiongehalts der Zellen im Vergleich zu Kontrollzellen, zur Bestimmung des Potentials, den zellulären Glutathiongehalt zu senken und
b2) Färben der Zellen mit zumindest einem Fluoreszenz- oder Chemilumineszenzfarbstoff, insbesondere mit 2',7' Dichlorofluorescein (H₂ DCFDA), zur Bestimmung des Potentials, ROS zu induzieren.

2. Verfahren nach Anspruch 1, **dadurch gekennzeichnet, dass** das Verfahren *in vitro* erfolgt.

3. Verfahren nach Anspruch 1, **dadurch gekennzeichnet, dass** das Verfahren *exvivo* erfolgt.

## Claims

1. A method for determining the potential of pharmaceutical substances to lower the cellular glutathione content and to raise the cellular ROS content, thus determining the potential of the pharmaceutical substances to be used in the therapy of T-cell-mediated autoimmune diseases, comprising the following steps:
a) incubation of cells with different concentrations of the given pharmaceutical substance to be tested; and
b1) determination of the glutathione content of the cells in comparison to control cells, for determining the potential to lower the cellular glutathione content and
b2) staining of the cells with at least one fluorescent or chemiluminescent dye, in particular with 2',7' dichlorofluorescein (H₂ DCFDA), for determining the potential to induce ROS.

2. The method as claimed in claim 1, **characterized in that** the method is implemented *in vitro.*

3. The method as claimed in claim 1, **characterized in that** the method is implemented *ex vivo.*

## Revendications

1. Procédé pour la détermination du potentiel de substances pharmaceutiques à abaisser la teneur en glutathion cellulaire et à augmenter la teneur en dérivés réactifs de l'oxygène (DRO) cellulaires, et donc du potentiel des substances pharmaceutiques à être utilisées dans la thérapie de maladies auto-immunes induites par les lymphocytes T, comportant les étapes suivantes :
a) incubation de cellules avec différentes concentrations de la substance pharmaceutique respective à tester ; et
b1) détermination de la teneur en glutathion des cellules en comparaison de cellules témoins, pour la détermination du potentiel à abaisser la teneur en glutathion cellulaire et
b2) coloration des cellules avec au moins un colorant fluorescent ou chimiluminescent, en particulier avec de la 2',7'-dichlorofluorescéine (H₂ DCFDA), pour la détermination du potentiel à induire des DRO.

2. Procédé selon la revendication 1, **caractérisé en ce que** le procédé s'effectue in vitro.

3. Procédé selon la revendication 1, **caractérisé en ce que** le procédé s'effectue ex vivo.
